(19) Europäisches Patentamt
European Patent Office
Office européen des brevets

(11) Veröffentlichungsnummer: **0 243 808 B1**

# (12) EUROPÄISCHE PATENTSCHRIFT

(45) Veröffentlichungstag der Patentschrift: **13.07.94**

(51) Int. Cl.[5]: **C07D 217/24**, A61K 31/47

(21) Anmeldenummer: **87105644.6**

(22) Anmeldetag: **16.04.87**

Die Akte enthält technische Angaben, die nach dem Eingang der Anmeldung eingereicht wurden und die nicht in dieser Patentschrift enthalten sind.

## (54) Isochinolindione, Verfahren zu ihrer Herstellung und diese Verbindungen enthaltende Arzneimittel.

(30) Priorität: **25.04.86 DE 3614000**

(43) Veröffentlichungstag der Anmeldung:
**04.11.87 Patentblatt 87/45**

(45) Bekanntmachung des Hinweises auf die Patenterteilung:
**13.07.94 Patentblatt 94/28**

(84) Benannte Vertragsstaaten:
**AT BE CH DE ES FR GB GR IT LI LU NL SE**

(56) Entgegenhaltungen:
**DE-A- 2 732 951**
**DE-A- 3 410 168**

(73) Patentinhaber: **BOEHRINGER MANNHEIM GMBH**

**D-68298 Mannheim(DE)**

(72) Erfinder: **Mertens, Alfred, Dr. rer. nat.**
**Beethovenstrasse 20**
**D-6905 Schriesheim(DE)**
Erfinder: **Müller-Beckmann, Bernd, Dr. med.vet.**
**Hochgewanne 46**
**D-6718 Grünstadt(DE)**

**EP 0 243 808 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Isochinolin-1,3-dione der allgemeinen Formel I

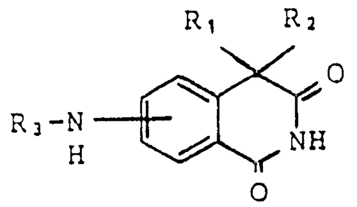

(I),

in welcher

$R_1$ ein Wasserstoffatom, eine $(C_1-C_6)$-Alkyl-, $(C_2-C_6)$-Alkenyl- oder - sofern $R_2$ ein Wasserstoffatom ist - eine verzweigte $(C_3-C_7)$-Alkyl- oder eine $(C_3-C_7)$-Cycloalkylgruppe bedeutet, die gegebenenfalls durch ein Heteroatom oder durch ein mit einer Alkylgruppe substituiertes Heteroatom unterbrochen ist,

$R_2$ ein Wasserstoffatom, eine $(C_1-C_6)$-Alkyl-, $(C_2-C_6)$-Alkenyl- oder zusammen mit $R_1$ eine $(C_3-C_8)$-Cycloalkyl-$(C_1-C_6)$-Alkyliden- und $(C_3-C_8)$-Cycloalkylidengruppe und

$R_3$ eine Gruppe

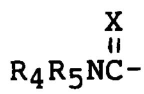

bedeutet, die in 5, 6, 7 oder 8-Stellung des Isochinolin-1,3-dions stehen kann und in der $R_4$ und $R_5$ gleich oder verschieden sein können und ein Wasserstoffatom, eine $(C_1-C_8)$-Alkyl-, $(C_1-C_3)$-Trihalogenalkyl, $(C_3-C_7)$-Cycloalkyl-, $(C_3-C_7)$-Cycloalkenyl-, Dialkylaminoalkyl-, Alkoxycarbonylalkyl-, Alkylcarbonylgruppe, wobei die Alkylreste 1-5 Kohlenstoffatome enthalten, eine Arylgruppe, wie eine substituierte Phenyl- oder Naphthylgruppe, oder eine Hetarylgruppe, wie eine substituierte Pyridyl- oder Pyrrolylgruppe, und X Sauerstoff, Schwefel oder die Gruppe N-$R_6$, in der $R_6$ ein Wasserstoffatom, eine $(C_1-C_3)$-Alkyl- oder Cyangruppe ist, darstellen,

Da die Verbindungen der allgemeinen Formel I für den Fall, daß $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenaggregation und verbessern die Mikrozirkulation.

In der DE-A-34 10 168 und in der DE-A-27 32 951 sind Isochinolin-dione beschrieben, die sich insbesondere durch einen zusätzlichen, ankondensierten Imidazolring von den hier beschriebenen, neuen Substanzen unterscheiden. Auch diese Verbindungen wirken kardiotonisch. Wie jedoch Vergleichsversuche zeigen, ist die Wirkung der neuen Verbindungen der vorliegenden Erfindung stärker als die der bekannten Verbindungen.

In der allgemeinen Formel I sind die Substituenten $R_1$ und $R_2$, die eine Alkyl- oder Alkenylgruppe bilden, geradkettig oder verzweigt. Insbesondere kommen für $R_1$ und $R_2$ Wasserstoff, die Methyl-, Ethyl- und Allylgruppe in Frage.

Stellt nur $R_2$ ein Wasserstoffatom dar, so sind als $R_1$, die Isopropyl-, 3- Pentyl-, Cyclopentyl-, Cyclohexyl-, Piperidyl- und N-Methylpiperidylgruppe bevorzugt.

Bilden $R_1$ und $R_2$ zusammen mit dem C-Atom, an das sie gebunden sind, einen Cycloalkylring, so handelt es sich dabei vorzugsweise um die Spirocyclopropyl-, Spirocyclobutyl-, Spirocyclopentyl-, Spirocyclohexyl- und Spirocyloheptylgruppe. Bilden $R_1$ und $R_2$ zusammen eine Alkylidengruppe und eine Cycloalkylidengruppe, so kommen insbesondere die Methyliden-, Ethyliden-, Isopropyliden-, Cyclopentyliden- und Cyclohexylidengruppe in Frage.

Die Gruppe

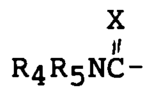

steht bevorzugt in der 6- oder 7- Stellung des Isochinolin-1,3-dions. Bilden die Substituenten $R_4$ und $R_5$ geradkettige oder verzweigte Alkylgruppen, so haben diese bevorzugt 1-5 C-Atome; bilden sie eine Trihalogenalkylgruppe, so ist die Trifluormethylgruppe bevorzugt; bilden sie Cycloalkyl- oder Cycloalkenylgruppen, so sind solche mit 5 oder 6 C-Atomen bevorzugt; bilden sie Dialkylaminoalkyl-, Alkoxycarbonylalkyl- und Alkylcarbonylgruppen, so sind Alkyl- und/oder Alkoxyreste mit 1-3 C-Atomen bevorzugt. Bildet X die Gruppe N-$R_6$ und ist $R_6$ eine Alkylgruppe, so besteht diese vorzugsweise aus einer Methyl-, Ethyl- oder Isopropylgruppe.

Die Herstellung der Verbindungen der allgemeinen Formel I kann zunaechst in der Weise erfolgen, daß man entweder

a) Amino-isochinolin-1,3-dione der allgemeinen Formel II

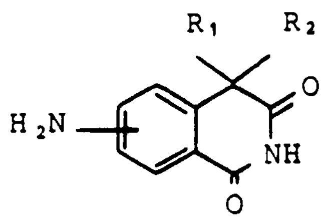

(II),

in der $R_1$ und $R_2$ die angegebene Bedeutung haben, mit einer Verbindung der allgemeinen Formel IIIa oder IIIb

$R_4$-N=C=X (IIIa)

$R_5$-N=C=X (IIIb) oder IV

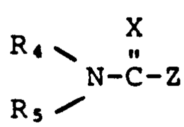

(IV),

in der $R_4$, $R_5$ und X die angegebene Bedeutung haben und Z eine abspaltbare Gruppe darstellt, zu Verbindungen der allgemeinen Formel I umsetzt,

oder

b) Verbindungen der allgemeinen Formel V

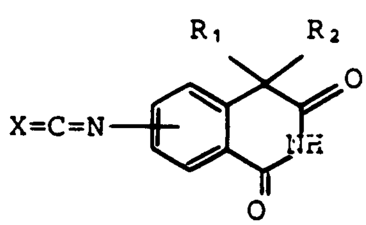

(V),

oder VI

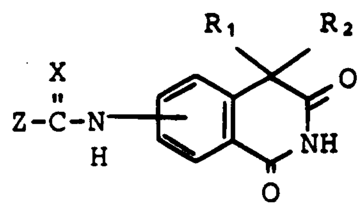

(VI),

in der $R_1$, $R_2$ und X die angegebene Bedeutung haben und Z eine abspaltbare Gruppe darstellt, mit Aminen der allgemeinen Formel VII

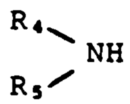

(VII),

in der $R_4$ und $R_5$ die angegebene Bedeutung haben, zu Verbindungen der allgemeinen Formel I umsetzt.

Eine abspaltbare Gruppe Z in Verbindungen der allgemeinen Formel IV oder VI ist in der Regel ein Halogenatom, wie z. B. ein Chlor-, Brom- und Jodatom, oder auch andere abspaltbare Reste wie z. B. der Mesylat-, Tosylat-, Phenoxy-, Methylmercapto- oder Phenylmercapto-Rest.

Umsetzungen von Verbindungen der allgemeinen Formel II mit Verbindungen der Formel III oder von Verbindungen der allgemeinen Formel V mit Verbindungen der Formel VII werden vorteilhaft in aprotischen Loesungsmitteln wie z.B. Methylenchlorid, Chloroform, Diethylether, Tetrahydrofuran, Dioxan, Toluol oder Dimethylformamid bei Temperaturen zwischen -70 ° C und 200 ° C vorzugsweise jedoch zwischen 0 ° C und 100 ° C durchgefuehrt.

Umsetzungen von Verbindungen der allgemeinen Formel II mit Verbindungen der Formel IV oder von Verbindungen der allgemeinen Formel VI mit Verbindungen der Formel VII koennen in Abhaengigkeit von der abspaltbaren Gruppe Z sowohl in protischen als auch in aprotischen Loesungsmitteln wie Wasser, Alkoholen, Methylenchlorid, Chloroform, Diethylether, Tetrahydrofuran, Dioxan, Toluol oder Dimethylformamid bei Temperaturen zwischen -70 ° C und 200 ° C vorzugsweise jedoch zwischen 0 ° C und 100 ° C durchgefuehrt werden.

Darueberhinaus koennen die Verbindungen der allgemeinen Formel I auch nachtraeglich in andere Verbindungen der allgemeinen Formel I umgewandelt werden, dies trifft zum Beispiel zu

a) fuer die Umwandlung von Verbindungen der allgemeinen Formel I, in der $R_1$, $R_2$ und $R_3$ die angegebene Bedeutung haben und X das Sauerstoffatom bedeutet, zu anderen Verbindungen der allgemeinen Formel I, in der X das Schwefelatom bedeutet, in dem man Verbindungen der Formel I mit einem das Schwefelatom uebertragenden Reagenz wie z.B. Phosphorpentasulfid oder 2,4-Bis-(4-methoxyphenyl)-2,4-dithioxo-1,3,2,4-dithiadiphosphetan in einem geeigneten Loesungsmittel wie z.B. Tetrahydrofuran, Dioxan, Ethylenglycoldimethylether, Benzol, Toluol oder Pyridin bei Temperaturen zwischen 0 ° C und der Siedetemperatur des Reaktionsgemisches umgesetzt,

b) fuer die Umsetzung von Verbindungen der allgemeinen Formel I, in der $R_3$ und X die angegebene Bedeutung haben und $R_1$ und $R_2$ Wasserstoff bedeuten, mit Verbindungen der allgemeinen Formel VIII

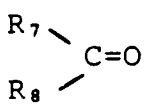

(VIII),

in welcher $R_7$ und $R_8$ Alkylgruppen sind oder $R_7$ zusammen mit $R_8$ eine $C_4$-$C_7$-Cycloalkylengruppe bildet, die gegebenenfalls durch ein Heteroatom oder durch ein mit einer Alkylgruppe substituiertes Heteroatom unterbrochen ist, in Gegenwart einer Base zu Verbindungen der allgemeinen Formel IX

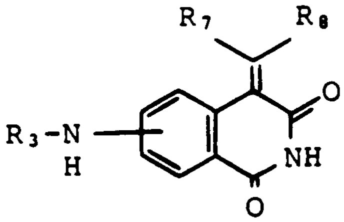

(IX),

in denen $R_7$ oder $R_8$ die oben angegebene Bedeutung besitzen, und anschließend Verbindungen der allgemeinen Formel IX durch katalytische Hydrierung in Verbindungen der allgemeinen Formel I, in denen $R_1$ oder $R_2$ gleich Wasserstoff ist, ueberfuehrt werden.

Die Kondensation von Isochinolin-1,3-dion mit einer Verbindung der Formel VIII geschieht in Gegenwart einer Base, vorzugsweise KOH oder NaOH.

Die katalytische Hydrierung von Verbindungen der Formel IX geschieht vorzugsweise mit Pd/C in alkoholischen Medien.

Ferner koennen die erhatenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol,Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure). Gelatine. Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere:

N-(4,4-Dimethyl-1,2,3,4-tetrahydro-1,3-dioxo-5-isochinolyinyl)harnstoff
N-Methyl-N-′(4,4-dimethyl-1,2,3,4-tetrahydro-1,3-dioxo-5-isochinolinyl)harnstoff
N-Propyl-N′-(4,4-dimethyl-1,2,3,4-tetrahydro-1,3-dioxo-5-isochinolinyl)harnstoff
N-(4,4-Dimethyl-1,2,3,4-tetrahydro-1,3-dioxo-6-isochinolinyl)harnstoff
N-Ethyl-N′-(4,4-dimethyl-1,2,3,4-tetrahydro-1,3-dioxo-6-isochinolinyl)harnstoff
N-Propyl-N′-(4,4-dimethyl-1,2,3,4-tetrahydro-1,3-dioxo-6-isochinolinyl)harnstoff
N-Methyl-N′-(4,4-diethyl-1,2,3,4-tetrahydro-1,3-dioxo-6-isochinolinyl)harnstoff
N-(1,2,3,4-tetrahydro-1,3-dioxo-7-isochinolinyl)harnstoff
N-Methyl-N′-(4-methyl-1,2,3,4-tetrahydro-1,3-dioxo-7-isochinolinyl)harnstoff
N-Methyl-N′-(4-allyl-1,2,3,4-tetrahydro-1,3-dioxo-7-isochinolinyl)thioharnstoff

N-Ethyl-N′-(4-cyclopentyl-1,2,3,4-tetrahydro-1,3-dioxo-7-isochinolinyl)harnstoff
N-Isopropyl-N′-[4-(1-methyl-4-piperidinyl)-1,2,3,4-tetrahydro-1,3-dioxo-7-isochinolinyl]harnstoff
N,N-Dimethyl-N′-(4,4-diethyl-1,2,3,4-tetrahydro-1,3-dioxo-7-isochinolinyl)harnstoff
N-Phenyl-N′-[spiro(cyclopentan-1,4′-1′,2′,3′,4′-tetrahydro-1′,3′-dioxo-7′-isochinolinyl)harnstoff
N-(4-Pyridyl)-N′-(4,4-diethyl-1,2,3,4-tetrahydro-1,3-dioxo-7-isochinolinyl)harnstoff
N-Acetyl-N′-[spiro(cyclopentan-1,4′-1′,2′,3′,4′-tetrahydro-1′,3′-dioxo-7′-isochinolinyl)]harnstoff
N-Cyclohexyl-N′-(4,4-dimethyl-1,2,3,4-tetrahydro-1,3-dioxo-7-isochinolinyl)harnstoff
N-(2-Cyclopenten-1-yl)-N′-(4,4-dimethyl-1,2,3,4-tetrahydro-1,3-dioxo-7-isochinolinyl)harnstoff
N-Methyl-N′-(4,4-dimethyl-1,2,3,4-tetrahydro-1,3-dioxo-7-isochinolinyl)guanidin
N-Cyano-N′-(4,4-dimethyl-1,2,3,4-tetrahydro-1,3-dioxo-7-isochinolinyl)guanidin
N-Methyl-N′-cyano-N″-(4,4-dimethyl-1,2,3,4-tetrahydro-1,3-dioxo-7-isochinolinyl)guanidin
N-Methyl-N′-(4,4-dimethyl-1,2,3,4-tetrahydro-1,3-dioxo-8-isochinolinyl)harnstoff
N-Ethyl-N′-(4,4-dimethyl-1,2,3,4-tetrahydro-1,3-dioxo-8-isochinolinyl)harnstoff
N-Propyl-N′-(4,4-dimethyl-1,2,3,4-tetrahydro-1,3-dioxo-8-isochinolinyl)harnstoff

Beispiel 1

N-(4,4-Dimethyl-1,2,3,4-tetrahydro-1,3-dioxo-7-isochinolinyl)harnstoff

1,3 g (6,3 mmol) 7-Amino-4,4-dimethyl-isochinolin-1,3 dion wurden in 30 ml 2 N Essigsaeure suspendiert, mit 1.0 g (12.5 mmol) Kaliumisocyanat versetzt und 6 Stunden bei 80 °C geruehrt. Der Ansatz wurde abgekuehlt, der Niederschlag abgesaugt und mit Wasser gewaschen. Der Rueckstand wurde in verduennter Natronlauge geloest, die Loesung mit verduennter Salzsaeure neutral gestellt, der Rueckstand abgesaugt und zur Reinigung mit Ethanol durchgearbeitet und abgesaugt.
Ausbeute: 0.6 g (38.5 %), Schmp. 260-64 °C.

Beispiel 2

N-Methyl-N′-(4,4-dimethyl-1,2,3,4-tetrahydro-1,3-dioxo-7-isochinolinyl)harnstoff

3 g (14.7 mmol) 7-Amino-4,4-dimethyl-isochinolin-1,3-dion wurden in 100 ml Methylenchlorid geloest, mit 5 ml Methylisocyanat unter Kuehlung versetzt und 2 Stunden bei 25 °C weitergeruehrt. Anschließend wurde der Ansatz eingeengt, gekuehlt, abgesaugt und aus Methanol umkristallisiert.
Ausbeute: 3.7 g (96.4 %), Schmp. 257-58 °C.

Analog zu Beispiel 2 erhaelt man

| | Bezeichnung | Ausbeute [%] | Schmp. [°C] Lsg.mittel |
|---|---|---|---|
| a) | N-Ethyl-N'-(4,4-dimethyl-1,2,3,4-tetrahydro-1,3-dioxo-7-isochinolinyl)harnstoff aus 7-Amino-4,4-dimethyl-isochinolin-1,3-dion und Ethylisocyanat | 75 | 225-28 Ethanol |
| b) | N-Isopropyl-N'-(4,4-dimethyl-1,2,3,4-tetrahydro-1,3-dioxo-7-isochinolinyl)harnstoff aus 7-Amino-4,4-dimethyl-isochinolin-1,3-dion und Isopropylisocyanat | 60.5 | 190-92 Essigester |
| c) | N-Phenyl-N'-(4,4-dimethyl-1,2,-3,4-tetrahydro-1,3-dioxo-7-isochinolinyl)harnstoff aus 7-Amino-4,4-dimethyl-isochinolin-1,3-dion und Phenylisocyanat | 83 | > 300 Ethanol |
| d) | N-Acetyl-N'-(4,4-dimethyl-1,2,-3,4-tetrahydro-1,3-dioxo-7-isochinolinyl)harnstoff aus 7-Amino-4,4-dimethyl-isochinolin-1,3-dion und Acetylisocyanat | 82.5 | 284-87 Methanol |
| e) | N-(2-Ethoxycarbonyl)ethyl-N'-(4,4-dimethyl-1,2,3,4-tetrahydro-1,3-dioxo-7-isochinolinyl)harnstoff aus 7-Amino-4,4-dimethyl-isochinolin-1,3-dion und (2-Ethoxycarbonyl)ethylisocyanat | 63.5 | 192-93 Ethanol |

| | Bezeichnung | Ausbeute [%] | Schmp. [°C] Lsg.mittel |
|---|---|---|---|
| f) | N-Methyl-N'-[spiro(cyclopentan-1,4'-1',2',3',4'-tetrahydro-1'-3'-dioxo-6'-isochinolinyl)]-harnstoff<br>aus<br>7'-Amino-spiro(cyclopentan-1,4'-2H',4H'-isochinolin)-1',3'-dion und Methylisocyanat | 91 | > 300 Ethanol |

Beispiel 3

N-Methyl-N'-(4,4-dimethyl-1,2,3,4-tetrahydro-1,3-dioxo-7-isochinolinyl)thioharnstoff

1.3 g (6.3 mmol) 7-Amino-4,4-dimethyl-isochinolin-1,3-dion wurden in 30 ml Chloroform geloest, mit 1.4 g Methylisothiocyanat versetzt und 6 Stunden am Rueckfluß gekocht. Der Ansatz wurde gekuehlt, der Niederschlag abgesaugt und aus Ethanol umkristallisiert.
Ausbeute: 0.8 g (46 %), Schmp. 206-07 °C.

Beispiel 4

N,N-Dimethyl-N'-(4,4-dimethyl-1,2,3,4-tetrahydro-1,3-dioxo-7-isochinolinyl)harnstoff

1.3 g (6.3 mmol) 7-Amino-4,4-dimethyl-isochinolin-1,3-dion wurden in 30 ml Pyridin geloest, unter Eiskuehlung mit 1.5 g Dimethylcarbamoylchlorid versetzt und 4 Stunden bei 25 °C weitergeruehrt. Anschließend wurde der Ansatz zur Trockne destilliert, der Rueckstand in Methylenchlorid aufgenommen, mit Wasser gewaschen, getrocknet und das Methylenchlorid abdestilliert. Der Rueckstand wurde aus Ethanol umkristallisiert.
Ausbeute: 1.1 g (63.6 %), Schmp. 256-58 °C.

Beispiel 5

N-(4-Pyridyl)-N'-(4,4-dimethyl-1,2,3,4-tetrahydro-1,3-dioxo-7-isochinolinyl)harnstoff x 1 mol HCl

1.5 g (7.34 mmol) 7-Amino-4,4-dimethyl-isochinolin-1,3-dion wurden in 40 ml Dioxan geloest, 3.6 g 4-Pyridincarbonsaeureazid zugesetzt und 4 Stunden bei 80 °C weitergeruehrt. Anschließend wurde das Loesungsmittel abdestilliert, der Rueckstand in Ethanol suspendiert, mit ethanolischer Salzsaeure versetzt und der Rueckstand abgesaugt. Der Rueckstand wurde in 2 N Natronlauge geloest, das nach kurzer Zeit ausfallende Natriumsalz abgesaugt und nach erneutem Suspendieren in Wasser mit 2 N Salzsaeure angesaeuert und abgesaugt.
Ausbeute: 1.6 g (60.4 %), Schmp. 255-59 °C als Hydrochlorid.

Beispiel 6

N-Cyano-N'-(4,4-dimethyl-1,2,3,4-tetrahydro-1,3-dioxo-7-isochinolinyl)guanidin

2 g (5.7 mmol) N-Cyano-N'-(4,4-dimethyl-1,2,3,4-tetrahydro-1,3-dioxo-7-isochinolinyl)-O-phenyl-isoharnstoff wurde in 100 ml Ethanol suspendiert und bei 80 °C Ammoniakgas eingeleitet. Nach 2 Stunden wurde der Ansatz gekuehlt, abgesaugt und der Rueckstand nochmals aus Ethanol umkristallisiert.
Ausbeute: 1.3 g (83.8 %), Schmp. 280-82 °C.

Das Ausgangsmaterial wurde wie folgt hergestellt:
5.5 g (23 mmol) N-Cyanodiphenoxyimidocarbonat und 4.7 g (23 mmol) 7-Amino-4,4-dimethyl-isochinolin-1,3-dion wurden in 110 ml Isopropanol 16 Stunden bei 50 °C geruehrt. Anschließend wurde der Ansatz gekuehlt, abgesaugt und mit Isopropanol gewaschen. Der Rueckstand wurde aus Ethanol umkristallisiert. Man erhaelt 4.1 g (51 %) N-Cyano-N′-(4,4-dimethyl-1,2,3,4-tetrahydro-1,3-dioxo-7-isochinolinyl)-O-phenyl-isoharnstoff vom Schmp. 214-16 °C.

Analog zu Beispiel 6 erhaelt man

| | Bezeichnung | Ausbeute [%] | Schmp. [°C] Lsg.mittel |
|---|---|---|---|
| a) | N-Cyano-N'-methyl-N"-(4,4-dimethyl-1,2,3,4-tetrahydro-1,3-dioxo-7-isochinolinyl)guanidin aus N-Cyano-N'-(4,4-dimethyl-1,2,3,4-tetrahydro-1,3-dioxo-7-isochinolinyl)-O-phenyl-isoharnstoff und 35proz. Methylamin-Loesung | 82.7 | 258-60 Methanol |

Beispiel 7

N-(2-Dimethylamino)ethyl-N′-(4,4-dimethyl-1,2,3,4-tetrahydro-1,3-dioxo-7-isochinolinyl)harnstoff

3.6 g (11.6 mmol) N-(2-Chlorethyl)-N′-(4,4-dimethyl-1,2,3,4-tetrahydro-1,3-dioxo-7-isochinolinyl)harnstoff wurden mit 30 ml 40proz. Dimethylaminloesung versetzt und 8 Stunden bei 30 °C geruehrt. Anschließend wurde das Loesungsmittel abdestilliert, der Rueckstand mit 2 N Salzsaeure durchgearbeitet und abgesaugt. Die waessrige saure Phase wurde neutral gestellt, mit Methylenchlorid extrahiert, getrocknet, eingeengt und der erhaltene Rueckstand aus Ethanol umkristallisiert.
Ausbeute: 1.9 g (51.5 %), Schmp. 204-05 °C.

Der als Ausgangsmaterial eingesetzte N-(2-Chlorethyl)-N′-(4,4-dimethyl-1,2,3,4-tetrahydro-1,3-dioxo-7-isochinolinyl)harnstoff wurde analog Beispiel 2 aus 7-Amino-4,4-dimethyl-isochinolin-1,3-dion und 2-Chlorethylisocyanat hergestellt.
Ausbeute: 90 %, Schmp. 177-79 °C aus Ethanol.

## Patentansprüche

**1.** Isochinolindion-Derivate der allgemeinen Formel I

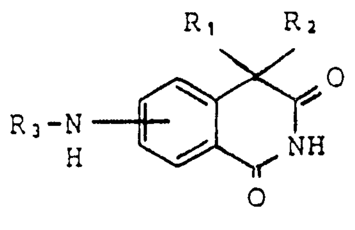

(I),

in welcher

$R_1$ ein Wasserstoffatom, eine ($C_1$-$C_6$)-Alkyl-, ($C_2$-$C_6$)-Alkenyl- oder - sofern $R_2$ ein Wasserstoffatom ist - eine verzweigte ($C_3$-$C_7$)-Alkyl- oder eine ($C_3$-$C_7$)-Cycloalkylgruppe bedeutet, die gegebenenfalls durch ein Heteroatom oder durch ein mit einer Alkylgruppe substituiertes Heteroatom unterbrochen ist,

$R_2$ ein Wasserstoffatom, eine ($C_1$-$C_6$)-Alkyl-, ($C_2$-$C_6$)-Alkenyl- oder zusammen mit $R_1$ eine ($C_3$-

$C_8$)-Cycloalkyl-($C_1$-$C_6$)-Alkyliden- und ($C_3$-$C_8$)-Cycloalkylidengruppe und

$R_3$ eine Gruppe

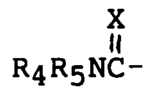

bedeutet, die in 5, 6, 7 oder 8-Stellung des Isochinolin-1,3-dions stehen kann und in der $R_4$ und $R_5$ gleich oder verschieden sein können und ein Wasserstoffatom, eine ($C_1$-$C_8$)-Alkyl-, ($C_1$-$C_3$)-Trihalogenalkyl, ($C_3$-$C_7$)-Cycloalkyl-, ($C_3$-$C_7$)-Cycloalkenyl-, Dialkylaminoalkyl-, Alkoxycarbonylalkyl-, Alkylcarbonylgruppe, wobei die Alkylreste 1-5 Kohlenstoffatome enthalten, eine Arylgruppe, wie eine substituierte Phenyl- oder Naphthylgruppe, oder eine Hetarylgruppe, wie eine substituierte Pyridyl- oder Pyrrolylgruppe, und X Sauerstoff, Schwefel oder die Gruppe N-$R_6$, in der $R_6$ ein Wasserstoffatom, eine ($C_1$-$C_3$)-Alkyl- oder Cyangruppe ist, darstellen,

sowie deren physiologisch verträglichen Salze.

**2.** Verbindungen der allgemeinen Formel I gemäß Anspruch 1, in der

$R_1$ und $R_2$ gleich oder verschieden sind und jeweils ein Wasserstoffatom, eine Methyl- oder Ethylgruppe oder $R_1$ und $R_2$ zusammen mit dem C-Atom, an das sie gebunden sind, eine Spirocyclopentyl- oder Spirocyclohexylgruppe bedeuten, und

$R_3$ eine Gruppe

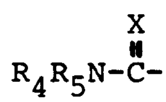

bedeutet, die in 6- oder 7-Stellung des Isochinolin-1,3-dions stehen kann und in der $R_4$ und $R_5$ gleich oder verschieden sein können und ein Wasserstoffatom, eine Methyl-, Ethyl-, Isopropyl-, Phenyl-, Pyridyl-, Acetyl-, Ethoxycarbonylethyl- und Dimethylaminoethylgruppe, und X Sauerstoff, Schwefel und die Gruppe N-$R_6$, in der $R_6$ eine Cyanogruppe ist, darstellen,

sowie deren physiologisch verträgliche Salze.

**3.** Verbindungen der allgemeinen Formel I gemäß Anspruch 1 oder 2 in der

$R_1$ und $R_2$ eine Methylgruppe oder $R_1$ und $R_2$ zusammen mit dem C-Atom, an das sie gebunden sind, eine Spirocyclopentylgruppe bedeuten, und

$R_3$ eine Gruppe

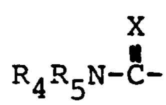

bedeutet, die in 7-Stellung des Isochinolin-1,3-dions steht und in der $R_4$ und $R_5$ gleich oder verschieden sein können und ein Wasserstoffatom oder eine Methylgruppe bedeuten, und X Sauerstoff und die Cyaniminogruppe darstellt,

sowie deren physiologisch verträgliche Salze.

**4.** Verfahren zur Herstellung von Isochinolindion-Derivaten der allgemeinen Formel I

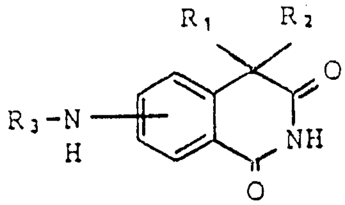

(I),

in welcher

$R_1$ ein Wasserstoffatom, eine $(C_1$-$C_6)$-Alkyl-, $(C_2$-$C_6)$-Alkenyl- oder - sofern $R_2$ ein Wasserstoffatom ist - eine verzweigte $(C_3$-$C_7)$-Alkyl- oder eine $(C_3$-$C_7)$-Cycloalkylgruppe bedeutet, die gegebenenfalls durch ein Heteroatom oder durch ein mit einer Alkylgruppe substituiertes Heteroatom unterbrochen ist,

$R_2$ ein Wasserstoffatom, eine $(C_1$-$C_6)$-Alkyl-, $(C_2$-$C_6)$-Alkenyl- oder zusammen mit $R_1$ eine $(C_3$-$C_8)$-Cycloalkyl-$(C_1$-$C_6)$-Alkyliden- und $(C_3$-$C_8)$-Cycloalkylidengruppe und

$R_3$ eine Gruppe

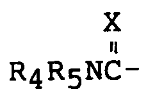

bedeutet, die in 5, 6, 7 oder 8-Stellung des Isochinolin-1,3-dions stehen kann und in der $R_4$ und $R_5$ gleich oder verschieden sein können und ein Wasserstoffatom, eine $(C_1$-$C_8)$-Alkyl-, $(C_1$-$C_3)$-Trihalogenalkyl, $(C_3$-$C_7)$-Cycloalkyl-, $(C_3$-$C_7)$-Cycloalkenyl-, Dialkylaminoalkyl-, Alkoxycarbonylalkyl-, Alkylcarbonylgruppe, wobei die Alkylreste 1-5 Kohlenstoffatome enthalten, eine Arylgruppe, wie eine substituierte Phenyl- oder Naphthylgruppe, oder eine Hetarylgruppe, wie eine substituierte Pyridyl- oder Pyrrolylgruppe, und X Sauerstoff, Schwefel oder die Gruppe N-$R_6$, in der $R_6$ ein Wasserstoffatom, eine $(C_1$-$C_3)$-Alkyl- oder Cyangruppe ist, darstellen,

sowie deren physiologisch verträglichen Salze, dadurch gekennzeichnet, daß man in an sich bekannter Weise

a) Amino-isochinolin-1,3-dione der allgemeinen Formel II

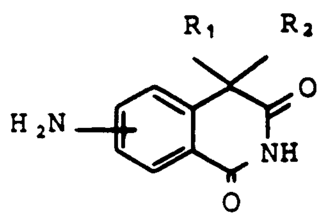

(II),

in der $R_1$ und $R_2$ die angegebene Bedeutung haben, mit einer Verbindung der allgemeinen Formel IIIa oder IIIb

$R_4$-N = C = X (IIIa)

$R_5$-N = C = X (IIIb)

oder IV

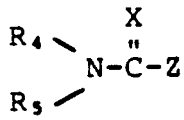

(IV),

in der $R_4$, $R_5$ und X die angegebene Bedeutung haben und Z eine abspaltbare Gruppe darstellt, zu Verbindungen der allgemeinen Formel I umsetzt,
oder
b) Verbindungen der allgemeinen Formel V

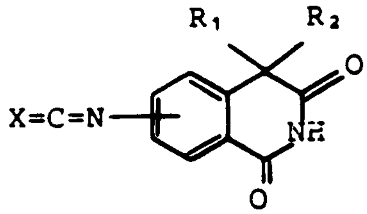

(V),

oder VI

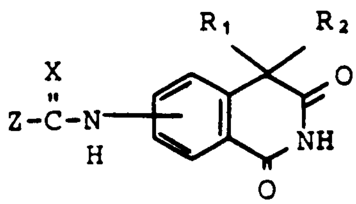

(VI),

in der $R_1$, $R_2$ und X die angegebene Bedeutung haben und Z eine abspaltbare Gruppe darstellt, mit Aminen der allgemeinen Formel VII

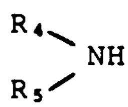

(VII),

in der $R_4$ und $R_5$ die angegebene Bedeutung haben, zu Verbindungen der allgemeinen Formel I umsetzt,
und anschließend gewünschtenfalls die erhaltenen Verbindungen in pharmakologisch unbedenkliche Salze überführt.

**5.** Verfahren zur Herstellung von Verbindungen der allgemeinen Formel I gemäß Anspruch 4, in der
$R_1$ und $R_2$ gleich oder verschieden sind und jeweils ein Wasserstoffatom, eine Methyl- oder Ethylgruppe oder $R_1$ und $R_2$ zusammen mit dem C-Atom, an das sie gebunden sind, eine Spirocyclopentyl- oder Spirocyclohexylgruppe bedeuten,
$R_3$ eine Gruppe

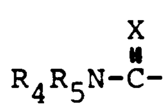

bedeutet, die in 6- oder 7-Stellung des Isochinolin-1,3-dions stehen kann und in der

$R_4$ und $R_5$ gleich oder verschieden sein können und ein Wasserstoffatom, eine Methyl-, Ethyl-, Isopropyl-, Phenyl-, Pyridyl-, Acetyl-, Ethoxycarbonylethyl-, und Dimethylaminoethylgruppe, und X Sauerstoff, Schwefel und die Gruppe $N-R_6$, in der $R_6$ eine Cyanogruppe ist, darstellen.

**6.** Verfahren zur Herstellung von Verbindungen der allgemeinen Formel I gemäß Anspruch 4 oder 5, in der

$R_1$ und $R_2$ eine Methylgruppe bedeutet oder zusammen mit dem C-Atom, an das sie gebunden sind, eine Spirocyclopentylgruppe darstellt,

$R_3$ eine Gruppe

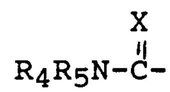

bedeutet, die in 7-Stellung des Isochinolin-1,3-dions steht und in der $R_4$ und $R_5$ gleich oder verschieden sein können und ein Wasserstoffatom oder eine Methylgruppe bedeuten, und X Sauerstoff und die Cyaniminogruppe darstellt.

**7.** Arzneimittel enthaltend mindestens eine Verbindung gemäß Ansprüche 1 - 3 sowie übliche Träger- und/oder Hilfsstoffe.

**8.** Verwendung von Verbindungen gemäß Ansprüche 1 - 3 zur Herstellung von Arzneimitteln zur Behandlung und/oder Prophylaxe von Herz- und Kreislauferkrankungen.

**9.** Verwendung von Verbindungen gemäß Ansprüche 1 - 3 zur Herstellung von Arzneimitteln.

## Claims

**1.** Isoquinolinedione derivatives of the general formula I

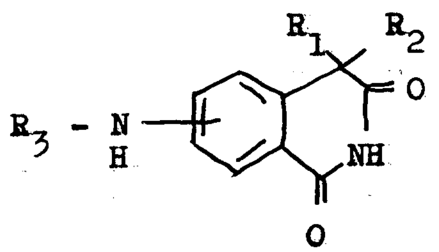

(I)

in which $R_1$ signifies a hydrogen atom, a $(C_1-C_6)$ alkyl, $(C_2-C_6)$-alkenyl or - insofar as $R_2$ is a hydrogen atom - a branched $(C_3-C_7)$alkyl or a $(C_3-C_7)$-cycloalkyl group which is possibly interrupted by a heteroatom or by a heteroatom substituted with an alkyl group, $R_2$ signifies a hydrogen atom, a $(C_1-C_6)$-alkyl, $(C_2-C_6)$-alkenyl or, together with $R_1$, a $(C_3-C_8)$-cycloalkyl-$(C_1-C_6)$-alkylidene and $(C_3-C_8)$-cycloalkylidene group and $R_3$ signifies a group

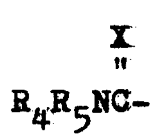

which can stand in the 5-, 6-, 7- or 8-position or the isoquinoline-1,3-dione and in which $R_4$ and $R_5$ can be the same or different and represent a hydrogen atom, a $(C_1-C_8)$-alkyl, $(C_1-C_3)$-trihaloalkyl, $(C_3-C_7)$-cycloalkyl, $(C_3-C_7)$-cycloalkenyl, dialkylaminoalkyl, alkoxycarbonylalkyl, alkylcarbonyl group, whereby the alkyl radicals contain 1 - 5 carbon atoms, an aryl group, such as a substituted phenyl or naphthyl group, or a hetaryl group, such as a substituted pyridyl or pyrrolyl group, and X represents oxygen,

sulphur or the group N-$R_6$, in which $R_6$ represents a hydrogen atom, a ($C_1$-$C_3$)-alkyl or cyano group, as well as their physiologically compatible salts.

**2.** Compounds of the general formula I according to claim 1, in which $R_1$ and $R_2$ are the same or different and in each case signify a hydrogen atom, a methyl, or ethyl group or $R_1$ and $R_2$, together with the C-atom to which they are attached, signify a spirocyclopentyl or spirocyclohexyl group and $R_3$ signifies a group

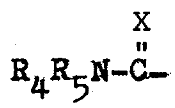

which can stand in the 6- or 7-position of the isoquinoline-1,3-dione and in which $R_4$ and $R_5$ can be the same or different and represent a hydrogen atom, a methyl, ethyl, isopropyl, phenyl, pyridyl, acetyl, ethoxycarbonylethyl and dimethylaminoethyl group and X represents oxygen, sulphur and the group N-$R_6$ in which $R_6$ is a cyano group; as well as their physiologically compatible salts.

**3.** Compounds of the general formula I according to claim 1 or 2, in which $R_1$ and $R_2$ signify a methyl group or $R_1$ and $R_2$, together with the carbon atom to which they are attached, signify a spirocyclopentyl group and $R_3$ signifies a group

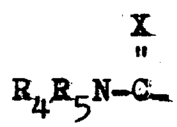

which stands in the 7-position of the isoquinoline-1,3-dione and in which $R_4$ and $R_5$ can be the same or different and signify a hydrogen atom or a methyl group and X represents an oxygen atom and the cyanimino group; as well as their physiologically compatible salts.

**4.** Process for the preparation of isoquinolinedione derivatives of the general formula I

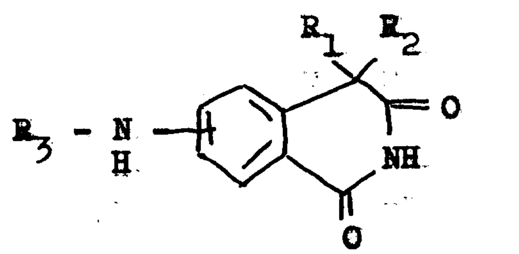

(I)

in which $R_1$ signifies a hydrogen atom, a ($C_1$-$C_6$)-alkyl, ($C_2$-$C_6$)-alkenyl or - insofar as $R_2$ is a hydrogen atom - a branched ($C_3$-$C_7$)alkyl or a ($C_3$-$C_7$)-cycloalkyl group which is possibly interrupted by a heteroatom or by a heteroatom substituted with an alkyl group, $R_2$ signifies a hydrogen atom, a ($C_1$-$C_6$)-alkyl, ($C_2$-$C_6$)-alkenyl or, together with $R_1$, a ($C_3$-$C_8$)-cycloalkyl-($C_1$-$C_6$)-alkylidene and ($C_3$-$C_8$)-cycloalkylidene group and $R_3$ signifies a group

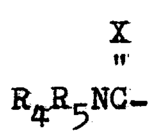

which can stand in the 5-, 6-, 7- or 8-position of the isoquinoline-1,3-dione and in which $R_4$ and $R_5$ can be the same or different and represent a hydrogen atom, a ($C_1$-$C_8$)alkyl, ($C_1$-$C_3$)-trihaloalkyl, ($C_3$-$C_7$)-cycloalkyl, ($C_3$-$C_7$)-cycloalkenyl, dialkylaminoalkyl, alkoxycarbonylalkyl, alkylcarbonyl group, whereby the alkyl radicals contain 1 - 5 carbon atoms, an aryl group, such as a substituted phenyl or naphthyl group or a hetaryl group, such as a substituted pyridyl or pyrrolyl group, and X represents oxygen,

sulphur or the group N-$R_6$, in which $R_6$ is a hydrogen atom, a $(C_1$-$C_3)$alkyl or cyano group, as well as of their physiologically compatible salts, characterised in that, in per se known way, one

a) reacts aminoisoquinoline-1,3-diones of the general formula II

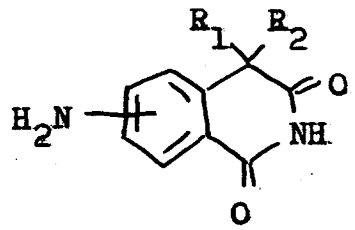

(II)

in which $R_1$ and $R_2$ have the given meanings, with a compound of the general formula IIIa or IIIb

$R_4$ - N = C = X (IIIa)

$R_5$ - N = C = X (IIIb)

or IV

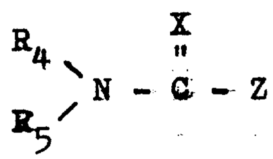

(IV)

in which $R_4$, $R_5$ and X have the given meaning and Z represents a group which can be split off, to give compounds of general formula I, or

b) reacts compounds of the general formula V

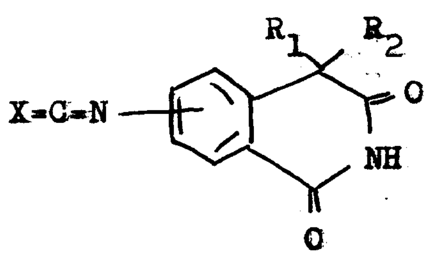

(V)

or VI

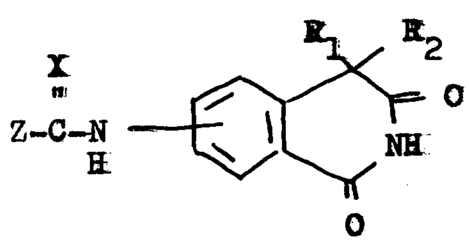

(VI)

in which $R_1$, $R_2$ and X have the given meanings, and Z represents a group which can be split off with amines of the general formula VII

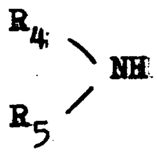

(VII)

in which $R_4$ and $R_5$ have the given meanings, to give compounds of the general formula I, and subsequently, if desired, converts the compounds obtained into pharmacologically compatible salts.

5. Process for the preparation of compounds of the general formula I according to claim 4 in which $R_1$ and $R_2$ are the same or different and in each case signify a hydrogen atom, a methyl or ethyl group or, $R_1$ and $R_2$ together with the C-atom to which they are attached, signify a spirocyclopentyl or spirocyclohexyl group, and $R_3$ signifies a group

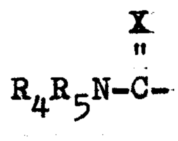

which can stand in the 6- or 7-position of the isoquinoline-1,3-dione and in which $R_4$ and $R_5$ can be the same or different and represent a hydrogen atom, a methyl, ethyl, isopropyl, phenyl, pyridyl, acetyl, ethoxycarbonylethyl and dimethylaminoethyl group and X is oxygen, sulphur or the group N-$R_6$, in which $R_6$ is a cyano group.

6. Process for the preparation of compounds of the general formula I according to claim 4 or 5 in which $R_1$ and $R_2$ signifies a methyl group or, together with the C-atom to which they are attached, represent a spirocyclopentyl group, $R_3$ signifies a group

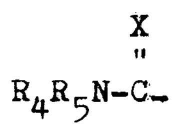

which stands in the 7-position of the isoquinoline-1,3-dione and in which $R_4$ and $R_5$ can be the same or different and signify a hydrogen atom or a methyl group and X represents oxygen and the cyan - imino group.

7. Medicaments containing at least one compound according to claims 1 - 3, as well as usual carrier and/or adjuvant materials.

8. Use of compounds according to claims 1 - 3 for the preparation of medicaments for the treatment and/or prophylaxis of heart and circulatory diseases.

9. Use of compounds according to claims 1 - 3 for the preparation of medicaments.

## Revendications

**1.** Dérivés d'isoquinoléinedione de formule générale I

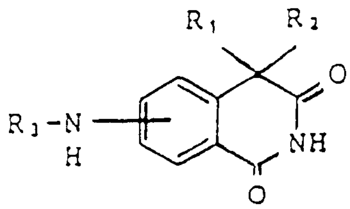 (I),

dans laquelle

$R_1$ représente un atome d'hydrogène, un groupe alkyle($C_1$-$C_6$), alcényle($C_2$-$C_6$) ou - dans le cas où $R_2$ représente un atome d'hydrogène - un groupe alkyle($C_3$-$C_7$) ramifié ou un groupe cycloalkyle($C_3$-$C_7$), qui peut être interrompu éventuellement par un hétéroatome ou par un hétéroatome portant un groupe alkyle,

$R_2$ représente un atome d'hydrogène, un groupe alkyle($C_1$-$C_6$), alcényle($C_2$-$C_6$) ou, conjointement avec $R_1$, un groupe cycloalkyl($C_3$-$C_8$)-alkylidène($C_1$-$C_6$) et un groupe cycloalkylidène-($C_3$-$C_8$), et

$R_3$ représente un groupe

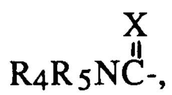

qui peut se trouver en position 5, 6, 7 ou 8 de l'isoquinoléine-1,3-dione, et $R_4$ et $R_5$ peuvent être identiques ou différents et représentent un atome d'hydrogène, un groupe alkyle($C_1$-$C_8$), trihalogénoalkyle($C_1$-$C_3$), cycloalkyle($C_3$-$C_7$), cycloalcényle($C_3$-$C_7$), dialkylaminoalkyle, alcoxycarbonylalkyle, alkylcarbonyle, les restes alkyle contenant 1-5 atomes de carbone, un groupe aryle tel qu'un groupe phényle ou naphtyle substitué, ou un groupe hétéroaryle tel qu'un groupe pyridyle ou pyrrolyle substitué, et X représente un atome d'oxygène, de soufre, ou le groupe N-$R_6$, dans lequel $R_6$ représente un atome d'hydrogène, un groupe alkyle($C_1$-$C_3$) ou un groupe cyano,

ainsi que leurs sels physiologiquement acceptables.

**2.** Composés de formule générale I selon la revendication 1, dans laquelle

$R_1$ et $R_2$ sont identiques ou différents et représentent chacun un atome d'hydrogène, un groupe méthyle ou éthyle, ou $R_1$ et $R_2$ forment, conjointement avec l'atome de C auquel ils sont liés, un groupe spirocyclopentyle ou spirocyclohexyle, et

$R_3$ représente un groupe

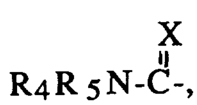

qui peut se trouver en position 6 ou 7 de l'isoquinoléine-1,3-dione et dans lequel $R_4$ et $R_5$ peuvent être identiques ou différents et représentent un atome d'hydrogène, un groupe méthyle, éthyle, isopropyle, phényle, pyridyle, acétyle, éthoxycarbonyléthyle ou diméthylaminoéthyle, et X représente un atome d'oxygène, de soufre et le groupe N-$R_6$, dans lequel $R_6$ représente un groupe cyano,

ainsi que leurs sels physiologiquement acceptables.

**3.** Composés de formule générale I selon la revendication 1 ou 2, dans laquelle

$R_1$ et $R_2$ représentent un groupe méthyle ou $R_1$ et $R_2$ forment, conjointement avec l'atome de C auquel ils sont liés, un groupe spirocyclopentyle, et

$R_3$ représente un groupe

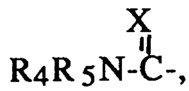

qui se trouve en position 7 de l'isoquinoléine-1,3-dione et dans lequel $R_4$ et $R_5$ peuvent être identiques ou différents et représentent un atome d'hydrogène ou un groupe méthyle, et X représente un atome d'oxygène ou un groupe cyanimino,

ainsi que leurs sels physiologiquement acceptables.

**4.** Procédé de préparation de dérivés d'isoquinoléinedione de formule générale I

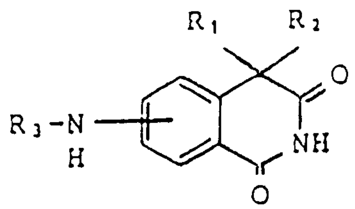

(I),

dans laquelle

$R_1$ représente un atome d'hydrogène, un groupe alkyle($C_1$-$C_6$), alcényle($C_2$-$C_6$) ou - dans le cas où $R_2$ représente un atome d'hydrogène - un groupe alkyle($C_3$-$C_7$) ramifié ou un groupe cycloalkyle($C_3$-$C_7$), qui peut être interrompu éventuellement par un hétéroatome ou par un hétéroatome portant un groupe alkyle,

$R_2$ représente un atome d'hydrogène, un groupe alkyle($C_1$-$C_6$), alcényle($C_2$-$C_6$) ou, conjointement avec $R_1$, un groupe cycloalkyl($C_3$-$C_8$)-alkylidène($C_1$-$C_6$) et un groupe cycloalkylidène-($C_3$-$C_8$), et

$R_3$ représente un groupe

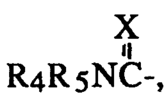

qui peut se trouver en position 5, 6, 7 ou 8 de l'isoquinoléine-1,3-dione, et $R_4$ et $R_5$ peuvent être identiques ou différents et représentent un atome d'hydrogène, un groupe alkyle($C_1$-$C_8$), trihalogénoalkyle($C_1$-$C_3$), cycloalkyle($C_3$-$C_7$), cycloalcényle($C_3$-$C_7$), dialkylaminoalkyle, alcoxycarbonylalkyle, alkylcarbonyle, les restes alkyle contenant 1-5 atomes de carbone, un groupe aryle tel qu'un groupe phényle ou naphtyle substitué, ou un groupe hétéroaryle tel qu'un groupe pyridyle ou pyrrolyle substitué, et X représente un atome d'oxygène, de soufre, ou le groupe N-$R_6$, dans lequel $R_6$ représente un atome d'hydrogène, un groupe alkyle($C_1$-$C_3$) ou un groupe cyano,

ainsi que leurs sels physiologiquement acceptables, caractérisé en ce que de manière connue en soi

a) on fait réagir une amino-isoquinoléine-1,3-dione de formule générale II

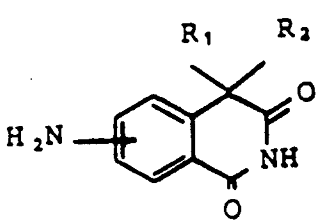

(II)

dans laquelle $R_1$ et $R_2$ ont les significations mentionnées, avec un composé de formule générale IIIa ou IIIb

$R_4$-N=C=X (IIIa)

$R_5$-N=C=X (IIIb)

ou IV

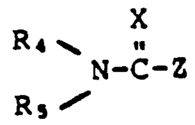

(IV)

dans laquelle $R_4$, $R_5$ et X ont les significations mentionnées et Z représente un groupe se séparant facilement, pour obtenir des composés de formule générale I,
ou
b) on fait réagir des composés de formule générale V

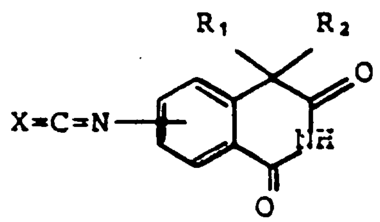

(V)

ou VI

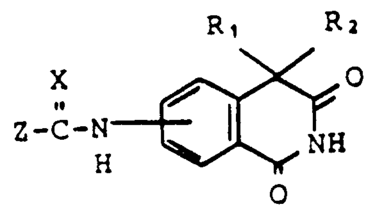

(VI)

dans laquelle $R_1$, $R_2$ et X ont les significations mentionnées et Z représente un groupe se séparant facilement, avec des amines de formule générale VII

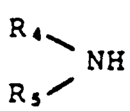

(VII)

dans laquelle $R_4$ et $R_5$ ont les significations mentionnées, pour obtenir des composés de formule générale I,

et ensuite, si on le souhaite, on transforme les composés obtenus en leurs sels pharmacologiquement acceptables.

**5.** Procédé de préparation de composés de formule générale I selon la revendication 4, dans laquelle

$R_1$ et $R_2$ sont identiques ou différents et représentent chacun un atome d'hydrogène, un groupe méthyle ou éthyle, ou $R_1$ et $R_2$ forment, conjointement avec l'atome de C auquel ils sont liés, un groupe spirocyclopentyle ou spirocyclohexyle,

$R_3$ représente un groupe

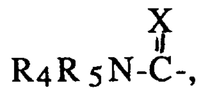

qui peut se trouver en position 6 ou 7 de l'isoquiinoléine-1,3-dione et $R_4$ et $R_5$ peuvent être identiques ou différents et représentent un atome d'hydrogène, un groupe méthyle, éthyle, isopropyle, phényle, pyridyle, acétyle, éthoxycarbonyléthyle ou diméthylaminoéthyle, et X représente un atome d'oxygène, de soufre, et le groupe N-$R_6$ dans lequel $R_6$ représente un groupe cyano.

**6.** Procédé de préparation de composés de formule générale I selon la revendication 4 ou 5, dans laquelle

$R_1$ et $R_2$ représentent un groupe méthyle ou forment, conjointement avec l'atome de C auquel ils sont liés, un groupe spirocyclopentyle,

$R_3$ représente un groupe

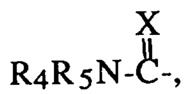

qui se trouve en position 7 de l'isoquinoléine-1,3-dione et dans lequel $R_4$ et $R_5$ sont identiques ou différents et représentent un atome d'hydrogène ou un groupe méthyle, et X représente un atome d'oxygène et un groupe cyanimino.

**7.** Médicament contenant au moins un composé selon les revendications 1 - 3, ainsi que des véhicules et/ou adjuvants usuels.

**8.** Utilisation des composés selon les revendications 1 - 3, pour la préparation de médicaments destinés au traitement et/ou à la prévention de maladies cardiovasculaires.

**9.** Utilisation des composés selon les revendications 1 - 3 pour la préparation de médicaments.